(19) **Europäisches Patentamt / European Patent Office / Office européen des brevets**

(11) **EP 1 521 771 B1**

(12) # EUROPEAN PATENT SPECIFICATION

(45) Date of publication and mention
of the grant of the patent:
**24.09.2008 Bulletin 2008/39**

(51) Int Cl.:
*C07K 14/315* (2006.01)   *C07K 2/00* (2006.01)
*C07K 16/12* (2006.01)   *C12N 15/11* (2006.01)
*C12N 15/63* (2006.01)   *C12N 5/00* (2006.01)
*G01N 33/50* (2006.01)

(21) Application number: **03764076.0**

(22) Date of filing: **11.07.2003**

(86) International application number:
**PCT/IB2003/003397**

(87) International publication number:
**WO 2004/007541 (22.01.2004 Gazette 2004/04)**

(54) **STREPTOCOCCUS PNEUMONIAE PBP2x MINI-PROTEIN AND USES THEREOF.**

PBP2X MINI-PROTEIN VON STREPTOCOCCUS PNEUMONIAE UND DESSEN VERWENDUNGEN

MINI-PROTEINE PBP2X DE STREPTOCOCCUS PNEUMONIAE ET UTILISATIONS
CORRESPONDANTES

(84) Designated Contracting States:
**AT BE BG CH CY CZ DE DK EE ES FI FR GB GR
HU IE IT LI LU MC NL PT RO SE SI SK TR**

(30) Priority: **11.07.2002 FR 0208724**

(43) Date of publication of application:
**13.04.2005 Bulletin 2005/15**

(73) Proprietors:
• **COMMISSARIAT A L'ENERGIE ATOMIQUE**
**75015 Paris (FR)**
• **CENTRE NATIONAL DE
LA RECHERCHE SCIENTIFIQUE (CNRS)
75794 Paris Cedex 16 (FR)**

(72) Inventors:
• **DIDEBERG, Otto
F-38100 Grenoble (FR)**
• **VERNET, Thierry
F-38000 Grenoble (FR)**
• **MOUZ, Nicolas
38760 SAINT PAUL DE VARCES (FR)**

(74) Representative: **Vialle-Presles, Marie José et al
Cabinet ORES,
36,rue de St Pétersbourg
75008 Paris (FR)**

(56) References cited:
**WO-A-98/48041**

• **GORDON E ET AL: "The crystal structure of the penicillin-binding protein 2x from Streptococcus pneumoniae and its acyl-enzyme form: Implication in drug resistance." JOURNAL OF MOLECULAR BIOLOGY, vol. 299, no. 2, 2000, pages 477-485, XP002240852 ISSN: 0022-2836 cited in the application**
• **LAIBLE ET AL: "Nucleotide sequences of the pbpX genes encoding the penicillin-binding proteins 2x from Streptococcus pneumoniae R6 and a cefotaxime-resistant mutant, C506" MOLECULAR MICROBIOLOGY, BLACKWELL SCIENTIFIC, OXFORD, GB, vol. 3, no. 10, 1989, pages 1337-1348, XP002117223 ISSN: 0950-382X cited in the application**
• **PARES S ET AL.: "X-ray structure of Streptococcus pneumoniae PBP2x, a primary penicillin target enzyme" NATURE STRUCTURAL BIOLOGY, vol. 3, no. 3, March 1996 (1996-03), pages 284-289, XP009010741 cited in the application**
• **DESSEN ANDREA ET AL: "Crystal structure of PBP2x from a highly penicillin-resistant Streptococcus pneumoniae clinical isolate. A mosaic framework containing 83 mutations." JOURNAL OF BIOLOGICAL CHEMISTRY, vol. 276, no. 48, 30 November 2001 (2001-11-30), pages 45106-45112, XP002240853 November 30, 2001 ISSN: 0021-9258 cited in the application**

- MOUZ NICOLAS ET AL: "Mutations in the active site of penicillin-binding protein PBP2x from Streptococcus pneumoniae: Role in the specificity for beta-lactam antibiotics." JOURNAL OF BIOLOGICAL CHEMISTRY, vol. 274, no. 27, 2 July 1999 (1999-07-02), pages 19175-19180, XP002240854 ISSN: 0021-9258 cited in the application
- MOUZ N ET AL: "Identification of a structural determinant for resistance to beta-lactam antibiotics in Gram-positive bacteria." PROCEEDINGS OF THE NATIONAL ACADEMY OF SCIENCES OF THE UNITED STATES, vol. 95, no. 23, 10 November 1998 (1998-11-10), pages 13403-13406, XP002240855 Nov. 10, 1998 ISSN: 0027-8424 cited in the application
- ASAHI Y ET AL: "Diversity of substitutions within or adjacent to conserved amino acid motifs of penicillin-binding protein 2X in cephalosporin-resistant Streptococcus pneumoniae isolates." ANTIMICROBIAL AGENTS AND CHEMOTHERAPY. UNITED STATES MAY 1999, vol. 43, no. 5, May 1999 (1999-05), pages 1252-1255, XP002240856 ISSN: 0066-4804
- JHOTI H: "High-throughput structural proteomics using x-rays" TRENDS IN BIOTECHNOLOGY, ELSEVIER PUBLICATIONS, CAMBRIDGE, GB, vol. 19, no. 10, 1 October 2001 (2001-10-01), pages S67-S71, XP004310381 ISSN: 0167-7799

**Description**

The present invention relates to a modified recombinant protein derived from *Streptococcus pneumoniae* PBP2x, referred to as mini-PBP2x, and also to uses thereof for selecting and identifying antibiotics which are active on β-lactam-resistant strains of *S. pneumoniae.*

Resistance to antibiotics represents a major problem in anti-infection therapy. For several years, the appearance of an increasing number of bacterial strains very resistant to the compounds of the β-lactam family (penicillins and cephalosporins, etc.), which represent the antibiotics most commonly used throughout the world for more than 60 years, has been observed; currently, 21% of clinical isolates of *Streptococcus pneumoniae,* one of the major pathogens of the upper respiratory pathways, are very resistant to β-lactams (minimum inhibitory concentration (MIC) > 2 μg/ml; Doern et al., Antimicrob. Agents Chemother, 2001, 45, 1721-1729).

The target for β-lactams is PBPs (Penicillin Binding Proteins), membrane proteins which catalyse essential steps of the synthesis of bacterial wall peptidoglycan. Each bacterial species has several PBPs, the molecular weight of which varies between 30 kDa and 100 kDa.

The high molecular weight PBPs comprise a short cytoplasmic domain, a single transmembrane domain and a large periplasmic domain, and are divided up into class A (having both transpeptidase activity and glyco-syltransferase activity (bridging)) and into class B (having an N-terminal domain of unknown function and a domain where the transpeptidase activity is located), which are readily identifiable by virtue of units of amino acids which are conserved for each class of PBP.

The low molecular weight PBPs essentially possess carboxypeptidase activity. Biochemical studies indicate that only the high molecular weight PBPs are multifunctional enzymes essential to bacterial survival; on the other hand, the low molecular weight PBPs are not essential and regulate only the degree of bridging of the bacterial wall.

The method of action of β-lactams is based on structural analogy between the ring of β-lactams and the D-alanyl-D-alanine of the C-terminal end of the peptides of the peptidoglycan; β-lactams are pseudosubstrates for the transpeptidase, capable of acylating the serine residue of the active site of this transpeptidase, which is then deacylated very slowly, thus disturbing peptidoglycan synthesis.

The mechanisms of β-lactam resistance in Gram+ bacteria comprise essentially: production of β-lactamases which hydrolyse the ring of β-lactams before they reach their target (PBPs), alteration of membrane permeability, and modification of PBPs.

*Streptococcus pneumoniae* has developed β-lactam resistance by modifying its PBPs; the combination of point mutations in the PBPs genes and of events of homologous recombination of these genes with those of related *Streptococcus* strains (*S. mitis, S. oralis*) results in the production of PBPs which have a low affinity for β-lactams.

Among the *S. pneumoniae* PBPs, the PBP2x protein, which is essential for the survival of *S. pneumoniae* and constitutes the primary factor of resistance to β-lactams (Hakenbeck et al., J. Bacteriol., 1998, 180, 1831-1840), represents the major target for identifying novel antibiotics which are active on β-lactam-resistant strains of *S. pneumoniae.*

PBP2x is a 750 amino acid protein comprising a cytoplasmic region (positions 1 to 18), a transmembrane region (positions 19 to 48), a non penicillin-binding domain (or n-PB, positions 49 - 265), a penicillin-binding domain/transpeptidase domain (positions 266 to 615) and a C-terminal domain (616 - 750).

Structure-function analysis of PBP2x has made it possible to specify the molecular mechanisms of the resistance of PBP2x to β-lactams (Mouz et al., P.N.A.S., 1998, 95, 13403 - 13406; J. Biol. Chem., 1999, 274, 19175-19180).

The 3-dimensional structure of PBP2x has been determined from a PBP2x comprising a deletion of the cytoplasmic and transmembrane regions, referred to as PBP2x*; this structure has been determined with a resolution of, respectively, 3.5, 2.4 and 3.2 Å, for a β-lactam-sensitive strain of *S. pneumoniae* (strain R6: Pares et al., Nature Struct. Biol., 1996, 3, 284 - 289; Gordon et al., J. Mol. Biol., 2000, 299, 477 - 485) and for a resistant clinical isolate (Dessen et al., J. Biol. Chem., 2001, 276, 45106 - 45112; accession numbers in the database PROTEIN DATA BANK (http://www.rcsb.org/), respectively 1PMD, 1QME and 1K25).

The three-dimensional structure of a complex between a PBP2x* of a sensitive strain (strain R6) and a β-lactam (cefuroxime) has also been determined with a resolution of 2.8 Å (accession number 1QMF in the database PROTEIN DATA BANK and Gordon et al., J. Mol. Biol., 2000, 299, 477-485).

However, the data currently available have not made it possible to identify novel antibiotic molecules which are active on β-lactam-resistant strains of *S. pneumoniae,* in particular due to the difficulty in obtaining crystals having a good diffraction power (2.5 Å) for native PBP2x (wild-type, sensitive to β-lactams), the PBP2x-inhibitor complexes and the β-lactam-resistant variants of PBP2x.

Specifically, analysis of PBP2x crystals which diffract at 2.4 Å (1QME, Gordon et al., J. Mol. Biol., 2000, 299, 477 - 485) has shown that they contain a form of PBP2x which is partially proteolysed at the peptide bond between the residues of positions 182 and 183, obtained under non-reproducible experimental conditions, resulting from exposure of the PBP2x protein at 25°C for several months, during the crystallization process.

The inventors have investigated, choosing, as a model, PBP2x of a penicillin-sensitive strain of *S. pneumoniae* (strain R6), the 3-dimensional structure of which is known, whether it is possible to obtain PBP2xs which are more crystallizable in a reproducible manner and which have a better diffraction power.

They have thus noted that additional deletions in the region corresponding to the n-PB domain (positions 50 to 265 of PBP2x) which do not modify the enzymatic properties of PBP2x (binding to β-lactams and - transpeptidase activity) make it possible to readily obtain crystals which have a better quality of diffraction.

The approach envisaged can be applied to any PBP2x derived from a β-lactam-sensitive or -resistant strain of *S. pneumoniae*.

This mini-protein represents a tool which is perfectly suited to selecting and identifying novel antibiotics which are active on β-lactam-resistant strains of *S. pneumoniae.*

Consequently, a subject of the present invention is a protein derived from a *Streptococcus pneumoniae* PBP2x, characterized in that it consists of a concatenation of the fragments corresponding respectively to the amino acids located between positions 74 to 90, 186 to 199, 218 to 228 and 257 - 750, with reference to the sequence of the PBP2x protein of the strain R6 (SWISSPROT P14677 or GENBANK 18266817), each one of said fragments being preceded by a peptide fragment of 1 to 7 amino acids.

The protein according to the invention is hereinafter referred to as PBP2x mini-protein or mini-PBP2x; it therefore comprises the deletion of amino acids located respectively between positions 1 to 73, 91 to 185, 200 to 217 and 229 to 256, as defined above, and the insertion, in place of said deletions, of a peptide fragment of 1 to 7 amino acids.

According to an advantageous embodiment of said mini-PBP2x protein, said peptide fragment of 1 to 7 amino acids comprises amino acids of the sequence of said *Streptococcus pneumoniae* PBP2x protein corresponding to those located between positions -1 to -7, relative to the residues of positions 74, 186, 218 and 257, and/or between positions +1 to +7, relative to the residues of positions 90, 199 and 228, as defined above.

According to another advantageous embodiment of said mini-PBP2x protein, said peptide fragment of 1 to 7 amino acids comprises amino acids in which the volume of the side chain is small, such as alanine (A), serine (S), glycine (G) or threonine (T).

For the purpose of the present invention, said *S. pneumoniae* PBP2x protein is defined by the following characteristics:

• it is encoded by the gene referred to as *pbpX*, corresponding to that located in the genome of the *S. pneumoniae* strain R6, between positions 2263 and 4515 of the locus having the NCBI accession number AE008411 or the GENBANK accession number 15457852;

it comprises the following amino acid units (one-letter code), specific to the class B PBPs:

| | |
|---|---|
| M1: | RGXhX(D/S)RSGXXXA |
| M2: | (R/K)XX**P**XG |
| M3: | (G/Y)h**E**XXXDXXL |
| M4: | hXX(S/T)hDXXX**Q** |
| M5: | T(G/S)EhhXXXXSPXh(D/N) |
| M6: | hEP(A/G)**S**XX**K** |
| M7: | hXX**S**XNh |
| M8: | **K**(**T**/S)**G**, |

in which the amino acids in bold are strictly conserved in the sequences of class B PBPs; / represents an alternative, for example D/S represents an aspartic acid or a serine; X represents any amino acid; h represents a hydrophobic amino acid and the other letters represent the amino acids most commonly encountered at this position; and

its sequence exhibits, over its entirety, at least 30% identity, preferably at least 50% identity, or at least 85% similarity, with the sequence of the strain R6 (SWISSPROT P14677).

The identity of a sequence relative to a reference sequence is assessed as a function of the percentage of amino acids residues which are identical, when the two sequences are aligned, so as to obtain the maximum correspondence between them.

A protein which has an amino acid sequence having at least X% identity with a reference sequence is defined, in the present invention, as a protein whose sequence can include up to 100-X alterations per 100 amino acids of the reference sequence, while at the same time conserving the functional properties of said reference protein. For the purpose of the present invention, the term "alteration" includes deletions, substitutions or insertions, which are consecutive or dispersed, of amino acids in the reference sequence.

The similarity of a sequence relative to a reference sequence is assessed as a function of the percentage of amino acid residues which are identical or which differ by conservative substitutions, when the two sequences are aligned so as to obtain the maximum correspondence between them. For the purpose of the present invention, the term "conservative substitution" is intended to mean the substitution of an amino acid with another which has similar chemical properties (size, charge or polarity), which generally does not modify the functional properties of the protein.

A protein which has an amino acid sequence having at least X% similarity with a reference sequence is defined, in the present invention, as a protein whose sequence can include up to 100-X nonconservative alterations per 100 amino acids of the reference sequence. For the purpose of the present invention, the term "nonconservative alterations" includes the deletions, nonconservative substitutions or insertions, which are consecutive or dispersed, of amino acids in the reference sequence.

The present invention encompasses the mini-PBP2xs derived from a PBP2x of any β-lactam-sensitive or -resistant strain of *S. pneumoniae*, in particular of β-lactam-resistant clinical isolates. By way of nonlimiting example, mention may be made of the β-lactam-resistant strain C 506, which is described in the article in the names of Laible et al. (Mol. Microbiol., 1989, 3, 1337-1348).

According to an advantageous embodiment of said mini-PBP2x protein, it is derived from a β-lactam-resistant strain of *Streptococcus pneumoniae.*

According to another advantageous embodiment of said mini-PBP2x protein, it consists of the concatenation of the fragments, as defined above, of PBP2x of the β-lactam-sensitive *Streptococcus pneumoniae* strain R6 (SWISSPROT P14677) and it has the sequence SEQ ID No. 1.

According to another advantageous embodiment of said mini-PBP2x protein, it comprises a substitution of at least one methionine residue with a selenomethionine residue.

According to another advantageous embodiment of said mini-PBP2x protein, it is associated with a ligand, in particular in the form of a mini-PBP2x/ligand complex.

In accordance with the invention, said ligand consists of an organic molecule, in particular a protein such as an antibody, or an inorganic molecule; said ligand is in particular a substrate, such as a pseudo-substrate, capable of binding to said mini-PBP2x protein via its active site and of inhibiting the activity of said mini-PBP2x.

According to yet another advantageous embodiment of said mini-PBP2x protein, it is in the form of a crystal.

In accordance with the invention, said crystal consists of a mini-PBP2x in free form or associated with a ligand as defined above.

In accordance with the invention, the conditions for crystallization of the mini-PBP2x are determined by the suspended drop technique. For example, crystals of mini-PBP2x are obtained under the following conditions: mini-PBP2x (12 mg/ml), 100 mM sodium Hepes, pH 7.5, 2% V/V PEG 400, 2M ammonium sulphate, at a temperature of 8°C.

The mini-PBP2xs consisting of fragments of PBP2x from β-lactam-resistant strains of *S. pneumoniae* are of use for screening and identifying novel antibiotics; the mini-PBP2xs consisting of fragments of PBP2x from sensitive strains of *S. pneumoniae*, in particular the mini-PBP2x of sequence SEQ ID No.1, are of use as a control, in screening and identifying antibiotics.

The subject of the present invention is also an isolated nucleic acid molecule, characterized in that it has a sequence encoding a mini-PBP2x as defined above, which are sense or antisense.

The nucleic acid molecules according to the invention are obtained by conventional methods, known in themselves, according to standard protocols such as those described in Current Protocols in Molecular Biology (Frederick M. AUSUBEL, 2000, Wiley and son Inc, Library of Congress, USA*).*

The sequences encoding PBP2x can be obtained by amplification of a nucleic acid sequence by PCR or RT-PCR, or else by screening genomic DNA libraries by hybridization with a homologous probe. For example, they are amplified by PCR using a suitable pair of primers, such as the pair of sequences SEQ ID Nos. 2-3.

The derived nucleic acid molecules, encoding a mini-PBP2x, are obtained by the conventional methods, making it possible to introduce mutations into a nucleic acid sequence, known in themselves, according to the abovementioned standard protocols. For example, the sequence encoding the mini-PBP2x can be obtained by site-directed mutagenesis according to the method of Kunkel et al. (P.N.A.S., 1985, 82, 488-492), using the primers SEQ ID Nos. 4 to 7, and then PCR amplification using the primers SEQ ID Nos. 8 and 9, as defined above.

A subject of the present invention is also a recombinant vector, characterized in that it comprises a nucleic acid molecule encoding a mini-PBP2x as defined above.

Preferably, said recombinant vector is an expression vector in which said nucleic acid molecule or one of its fragments is placed under the control of suitable elements for regulating transcription and translation. In addition, said vector may comprise sequences (tags) fused in-frame with the 5' and/or 3' end of said insert, of use for immobilizing and/or detecting and/or purifying the protein expressed from said vector.

Preferably, said expression vector is a prokaryotic vector.

These vectors are constructed and introduced into host cells by the conventional methods of recombinant DNA and genetic engineering, which are known in themselves.

A subject of the present invention is also cells transformed with a recombinant vector as defined above.

According to an advantageous embodiment of the invention, said cells are prokaryotic cells.

The recombinant vectors and the transformed cells as defined above are of particular use for producing mini-PBP2x and the derived peptides, as defined above.

A subject of the present invention is also the use of a mini-PBP2x as defined above, for screening and identifying antibiotics.

According to an advantageous embodiment of the invention, said screening is carried out by a method comprising at least the following steps:

$a_1$) bringing a mini-PBP2x as defined above into contact with a test substance,
$b_1$) detecting, by any suitable means, the binding of said test molecule with the mini-PBP2x and/or the inhibition of the activity of said mini-PBP2x resulting from this binding, and
$c_1$) selecting and identifying the active substances capable of binding to the mini-PBP2x and/or of inhibiting the activity of said mini-PBP2x, which can be used as antibiotics.

The binding of said test molecule with the mini-PBP2x can be measured using conventional binding assays which make it possible to detect molecules capable of binding covalently at the active serine ($S_{337}$, with reference to the sequence of PBP2x of the strain R6), in particular using a ligand pre-labelled with a chromophore or with a fluorophore, such as a cephalosporin coupled to a chromophore (nitrocefin), or else by measuring the decrease in intrinsic fluorescence of said mini-PBP2x, as described in the article in the names of Jamin et al., Biochem. J., 1993, 292, 735-741.

The inhibition of the enzymatic activity of the mini-PBP2x can be determined either by measuring the inhibition of hydrolysis of the thiol ester substrates, or by measuring the efficiency of acylation of the active serine, using conventional techniques as described in the article in the names of Zhao et al., J. Bacteriol., 1997, 179, 4901-4908.

According to another advantageous embodiment of the invention, said identification is carried out by a method comprising at least the following steps:

$a_2$) preparing crystals from a mini-PBP2x as defined above,
$b_2$) determining the three-dimensional structure of said mini-PBP2x from the crystal obtained in $a_2$), and
$c_2$) identifying active substances capable of binding to the mini-PBP2x and/or of inhibiting the activity of said mini-PBP2x, which can be used as antibiotics.

In accordance with the invention:

- the crystal is prepared from a free mini-PBP2x derived from a PBP2x from a β-lactam-sensitive or -resistant strain of *S. pneumoniae,* or else from mini-PBP2x/ligand complexes, by the suspended drop technique;
- the three-dimensional structure of the mini-PBP2x is determined by conventional techniques known in themselves, such as nuclear magnetic resonance and X-ray diffraction;
- the PBP2x inhibitors are identified by modelling of the structure of the free mini-PBP2x or of the mini-PBP2x/ligand complexes.

Advantageously:

- the mini-PBP2x according to step $a_2$) is subjected to a prior treatment comprising a step of denaturation by temperature or by a chemical agent, in the presence or absence of ligands, followed by a step of renaturation under suitable conditions;
- the mini-PBP2x according to step $a_1$) or $a_2$) consists of the concatenation of the fragments, as defined above, of a PBP2x from a β-lactam-resistant strain of *S. pneumoniae*; and
- the detection carried out in step $b_1$) or the determination and identification carried out respectively in steps $b_2$) and $c_2$) are carried out by comparison with a mini-PBP2x consisting of the concatenation of the fragments, as defined above, of a PBP2x from a β-lactam-sensitive strain of *S. pneumoniae.*

A subject of the invention is also a kit for implementing the methods as defined above, characterized in that it includes at least one protein as defined above.

The mini-PBP2x according to the invention, which can be produced in large amounts in a soluble functional form which is readily crystallizable, for all the PBP2xs, has the following advantages:

- it is suitable for systematic screening of novel antibiotic molecules using functional assays (high throughput screening),
- it is suitable for the structure-function study of the β-lactam-resistant variants of PBP2x and for the rational design of novel antibiotic molecules (molecular modelling or drug design).

Besides the arrangements above, the invention also comprises other arrangements, which will emerge from the following description, which refers to examples of use of the mini-PBP2x which is the subject of the present invention and also to Table I which summarizes the applicant's sequences and to the attached drawing, in which:

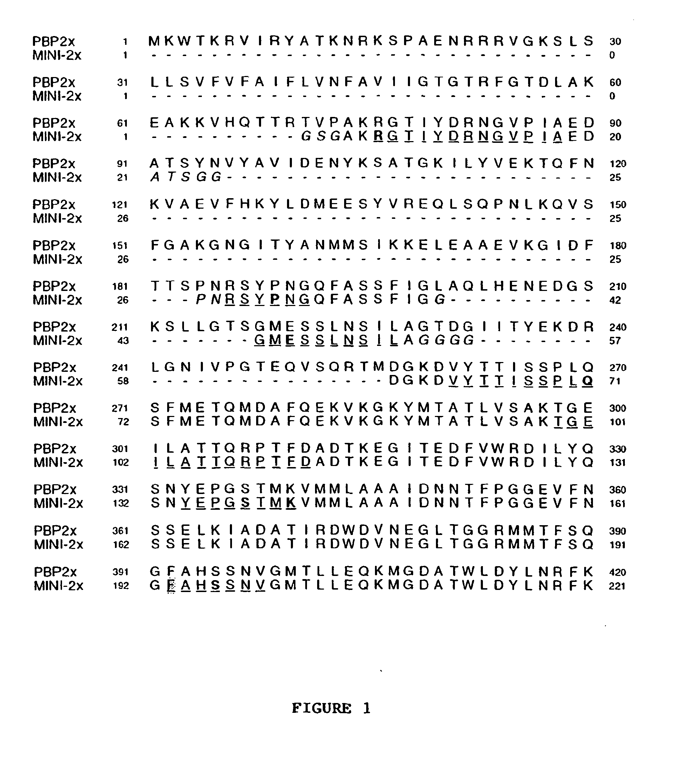

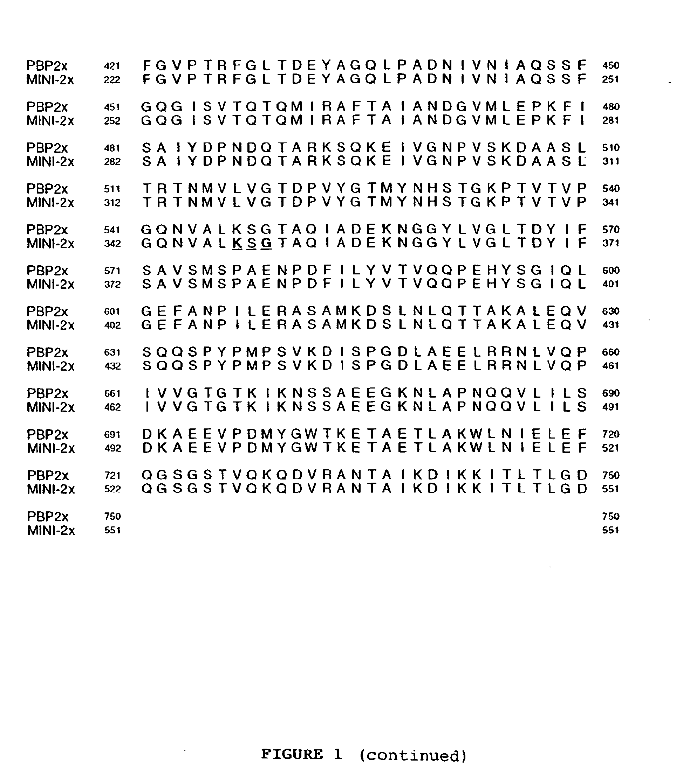

- Figure 1 illustrates the amino acid sequence of a mini-PBP2x (SEQ ID No. 1), derived from the PBP2x of the β-lactam-sensitive *S. pneumoniae* strain R6 (SWISSPROT P14677); the amino acids of PBP2x which have been deleted in the mini-PBP2x are replaced with a dash and those of the peptide fragments which have been inserted are represented in italics; the units specific to the class B PBPs are underlined.

**Table I: Sequence listing**

| Identification number | Sequence |
|---|---|
| SEQ ID No. 1 | Mini-PBP2x derived from PBP2x of the strain R6 (SWISSPROT P14677) |
| SEQ ID No. 2 | Primer 5'ICNter |
| SEQ ID No. 3 | Primer 3'ICCter |
| SEQ ID No. 4 | Oligonucleotide mini 1 |
| SEQ ID No. 5 | Oligonucleotide mini 2 |
| SEQ ID No. 6 | Oligonucleotide mini 3 |
| SEQ ID No. 7 | Oligonucleotide mini 4 |
| SEQ ID No. 8 | Oligonucleotide mini2x *NdeI* |
| SEQ ID No. 9 | Oligonucleotide mini2x *XhoI* |

It should be clearly understood, however, that these examples are given only by way of illustration of the subject of the invention, of which they in no way constitute a limitation.

## EXAMPLE 1: PRODUCTION OF A RECOMBINANT MINI-PBP2x

### 1) Construction of a vector for expression of a mini-PBP2x

a) Materials and methods

A vector for expression of a mini-PBP2x was constructed from the plasmid pGEX-S-PBP2x*-f1 (Mouz et al., J. Biol. Chem., 1999, 274, 19175-19180) containing the sequence encoding the PBP2x* of the β-lactam-sensitive *S. pneumoniae* strain R6 (fragment 49-750 of the PBP2x of the GENBANK amino acid sequence P14677, corresponding to the GENBANK nucleotide sequence X16367).

The deletions of the amino acids located between positions 49 to 73, 94 to 183, 200 to 217 and 230 to 256 and the insertion, in place of said deletions, respectively of the linking fragments Gly-Ser-Gly, Gly-Gly, Gly and Gly-Gly-Gly, was carried out by site-directed mutagenesis according to the method of Kunkel et al. (P.N.A.S., 1985, 82, 488-492), using the protocols as described in Mouz et al. (J. Biol. Chem., 1999, 274, 19175-19180).

More precisely, the phagemide called pGEX-S-PBP2x*-f1 is converted to single strand and then used as a matrix for the mutagenesis steps (deletions and insertions). The mutagenesis steps were carried out in two stages, the first with the oligonucleotides Mini 1 (SEQ ID No. 4) and Mini 3 (SEQ ID No. 6) and the second with the oligonucleotides Mini 2 (SEQ ID No. 5) and Mini 4 (SEQ ID No. 7):

- Mini 1:
5'-CATAAATAGTCCCACGTTTGGCCCCGGATCCACGCGGAACCAG-3', this oligonucleotide makes it possible to delete the nucleotide sequence corresponding to the amino acids located between positions 49 and 73 of PBP2x and to insert in place of said deletion a linking fragment corresponding to a peptide Gly-Ser-Gly.
- Mini 2:
5'-GTTTGGGTAACTACGATTGGGACCTCCAGAGGTTGCATCCTCAGCAATCGG-3', this oligonucleotide makes it possible to delete the nucleotide sequence corresponding to the amino acids located between positions 94 and 183 of PBP2x and to insert in place of said deletion a linking fragment corresponding to a peptide Gly-Gly.
- Mini 3:
5'-GTTCAAGGAACTCTCCATTCCACCGCCGATAAAACTAGAAGCAAATTG-3', this oligonucleotide makes it possible to delete the nucleotide sequence corresponding to the amino acids located between positions 200 and 217 of PBP2x and to insert in place of said deletion a linking fragment corresponding to a peptide Gly.
- Mini 4: 5'-TGTATAAACATCCTTACCGTCCCCACCTCCCCCCTGCAAGAATACTGTTC-3', this oligonucleotide

makes it possible to delete the nucleotide sequence corresponding to the amino acids located between positions 230 and 256 of PBP2x and to insert in place of said deletion a linking fragment corresponding to a peptide Gly-Gly-Gly.

The plasmid thus obtained, named pGEX-S-mini-PBP2x-f1, was used as a matrix to PCR amplify the DNA fragment corresponding to the mini-PBP2x and to introduce, at its ends, the *NdeI* and *XhoI* restriction sites, for cloning into the commercial vector pET30b (NOVAGEN). The oligonucleotides used for the PCR amplification are as follows:

- oligo Mini2x *NdeI* (SEQ ID No. 8):
  5'-CCGCATATGGCCAAACGTGGGACTATTTAT-3'
- oligo Mini2x *XhoI* (SEQ ID No. 9):
  5'-GGCTCGAGTTAGTCTCCTAAAGTTAATGTAAT-3'

The nucleotide sequence of the mini-PBP2x, in the expression vector thus obtained, named pET30b-mini-PBP2x, was confirmed by automatic sequencing.

b) Results

The peptide sequence deduced from the nucleotide sequence obtained by automatic sequencing has the sequence SEQ ID No. 1 (Figure 1), corresponding to that expected for a mini-PBP2x.

The mini-PBP2x consists of the succession of fragments corresponding respectively to the amino acids located between positions 74 to 93, 184 to 199, 218 to 229 and 257-750 of PBP2x (SWISSPROT accession number P14677), each fragment being preceded, respectively, by the linking fragment GSG, GG, G and GGG.

**2) Production and purification of mini-PBP2x**

a) Materials and methods

The mini-PBP2x is produced in *E. coli*, using the expression vector described in Example 1.1, and it is then purified by chromatography, successively on a Q-Sepharose, Resource Q and Superdex 200 column. The product obtained is analysed by polyacrylamide gel electrophoresis (SDS-PAGE) and by mass spectrometry (Electrospray ionization-mass spectrometry, ESI-MS).

More precisely, 2 litres of culture of the *E. coli* strain BL21 (DE 3), transformed with the plasmid pET30b-mini-PBP2x, are induced at an optical density of 1 (600 nm), for 15 h at 16°C. After centrifugation, the bacterial pellet is washed with 500 ml of buffer A (20 mM Tris HCl, pH 8.0, 20 mM NaCl, 1 mM EDTA) containing protease inhibitors, and is then resuspended in 50 ml of the same buffer.

After sonication (6 min on an ice/ethanol bed), the 50 ml of bacterial lysate are centrifuged at 40 000 g, at +4°C, for 20 min, and the supernatant obtained is loaded onto a 20 ml ion exchange column (Q-Sepharose, AMERSHAM-PHARMACIA), pre-equilibrated with buffer A. A linear sodium gradient is produced with buffer B (20 mM Tris HCl, pH 8.0, 300 mM NaCl, 1 mM EDTA). The mini-PBP2x protein is eluted from. 38% buffer B. The eluted fractions are analysed by Western blotting using rabbit polyclonal antibodies directed against PBP2x*, and are then pooled together. The pool of fractions containing the mini-PBP2x is diluted 10-fold in buffer A without NaCl, and is then loaded onto a 6 ml Resource Q column (AMERSHAM-PHARMACIA) pre-equilibrated with buffer A. A linear sodium gradient is produced with the buffer. The mini-PBP2x protein is eluted from 55% of buffer B. The fractions corresponding to the elution peak are analysed by SDS-15% PAGE. The purest fractions are pooled together and the pool obtained is concentrated to a volume of 2 to 4 ml, and then loaded onto a gel chromatography column (Superdex 200, 16/60, AMERSHAM-PHARMACIA), pre-equilibrated in a 10 mM Hepes buffer, pH 7.5, 100 mM NaCl, 1 mM EDTA. The mini-PBP2x is eluted in a symmetrical peak at an elution volume corresponding to an apparent mass of 60 kDa.

b) Results

The preparation of mini-PBP2x protein thus obtained has a degree of purity greater than 95%. The final yield of purified mini-PBP2x protein is 10 to 15 mg/litre of culture, depending on the preparations.

The purification by gel chromatography shows the presence of a symmetrical elution peak which reflects the homogeneity of the protein in terms of oligomerization. The elution volume corresponds to a monomeric protein.

The mini-PBP2x protein exhibits excellent solubility since it can be concentrated to 15 mg/ml without precipitating.

The analysis by mass spectrometry shows the presence of a homogeneous protein which has a molecular mass of 59 465 Da corresponding to that calculated from the amino acid sequence.

**EXAMPLE 2: ANALYSIS OF THE PHYSICOCHEMICAL AND ENZYMATIC PROPERTIES OF THE MINI-PBP2x**

**1) Materials and methods**

The physicochemical and enzymatic properties of the purified mini-PBP2x obtained in Example 1 were measured in a similar way to those of the variants of PBP2x, as described in Mouz et al. (P.N.A.S., 1998, 95, 13403-13406).
More precisely:

a) Molecular weight

The molecular weight was determined by ESI-MS (electrospray ionization-mass spectrometry).

b) Isoelectric point

The theoretical isoelectric point was determined from the amino acid composition of the mini-PBP2x.

c) Extinction coefficient

The molar extinction coefficient was determined experimentally by measuring inhibition of the mini-PBP2x by cefotaxime, or theoretically from the amino acid sequence.

d) Acylation kinetics

The efficiency of acylation of the mini-PBP2x by β-lactams, which is defined by the value $k_2/K$ where $K = k_{-1}/k$ according to equation 1 (Eql), was determined, in the presence of cefotaxime (3rd generation antibiotic); the interaction of the PBPs (enzyme E) with the β-lactams (I) is represented by the equation Eq1, in which EI, EI* and P represent, respectively, the Michaëlis-Menten complex, the acyl-enzyme covalent complex and the product (degraded β-lactam):

$$E + I \underset{k_{-1}}{\overset{k_1}{\longleftrightarrow}} EI \overset{k_2}{\rightarrow} EI^* \overset{k_3}{\rightarrow} E + P \quad (Eql).$$

e) Hydrolysis kinetics

The transpeptidase activity (hydrolytic activity) of the mini-PBP2x, defined by the value kcat/Km, was determined in the presence of two PBP2x substrate analogues [N-benzoyl-D-alanylmercaptoacetic thiol ester (S2d) and carboxymethylbenzoylaminothioacetate thiol ester (S2a)], synthesized according to the protocol described in Adam et al. (Biochem. J., 1990, 270, 525-529).

**2) Results**

The physicochemical characteristics of the mini-PBP2x are given in Table II below.

**TABLE II: Physicochemical parameters of the mini-PBP2x**

| Parameter | Mini-PBP2x |
|---|---|
| Molecular weight (daltons) | 59 465 $\pm$ 5 |
| Theoretical isoelectric point | 4.5 |
| Extinction coefficient at 280 nm ($mol^{-1}$ $cm^{-1}$ $L^{-1}$) | 52 830 $\pm$ 50 |

The kinetic parameters for the mini-PBP2x are given in Table III below, by comparison with those of PBP2x* (Jamin et al., Biochem. J., 1993, 292, 735-741; Mouz et al., P.N.A.S., 1998, 95, 13403-13406).

**Table III: Compared kinetic parameters of mini-PBP2x and of PBP2x***

| Parameters[1] | Mini-PBP2x | PBP2x*[2] | PBP2x*[3] |
|---|---|---|---|
| $k_2$/K (cefotaxime) | **146 100 $\pm$ 9 000** | 162 000 $\pm$ 400 | 209 000 + 1 800 |
| kcat/Km (S2a) | **76 $\pm$ 3** | 610 $\pm$ 150 | 139 $\pm$ 8 |
| kcat/Km (S2d) | **2 890 $\pm$ 400** | 5 000 $\pm$ 1 400 | 2 500 $\pm$ 200 |
| [1]Expressed as $M^{-1} \cdot s^{-1}$, [2]Jamin et al., Biochem. J., 1993, 292, 735-741, [3]Mouz et al., P.N.A.S., 1998, 95, 13403-13406 | | | |

Analysis of the kinetic parameters of the variants of PBP2x shows that, by comparison to PBP2x*, from which only the cytoplasmic and transmembrane domains of PBP2x are deleted (residues 1 to 48), the mini-PBP2x, which has additional deletions in the n-PB domain (non penicillin-binding domain), has enzymatic properties (binding to β-lactams and transpeptidase activity) equivalent to those of PBP2x*.

## EXAMPLE 3: CRYSTALLIZATION OF A MINI-PBP-2x

### 1) Materials and methods

The mini-PBP2x is purified as described in Example 1. The conditions for crystallization of the mini-PBP-2x, obtained by the suspended drop method, are as follows: mini-PBP2x (12 mg/ml), 100 mM sodium Hepes, pH 7.5, 2% V/V PEG 400, 2M ammonium sulphate, at a temperature of 8°C.

### 2) Results

The crystals appear after a few weeks; they exhibit a hexagonal bipyramidal form with a length of the order of 150 μm.

## EXAMPLE 4: ANALYSIS OF THE DIFFRACTION PROPERTIES OF A MINI-PBP2x

### 1) Materials and methods

The diffraction data was recorded at the ESRF (*European Synchrotron Radiation Facility*, Grenoble, France). More precisely, the crystals were immersed in the crystallization buffer as described in Example 3, supplemented with ethylene glycol (10% V/V) before being rapidly cooled in liquid nitrogen. The diffraction data was recorded on the ID14-EH2 beam line and processed using the MOSFLM and SCALA programs of the CCP4 suite (Acta Crystallograph. Sect. D, 1994, 54, 905-921).

### 2) Results

The crystals which diffract up to 2.5 Å exhibit a hexagonal elemental cell (a = b = 136.4 Å, c = 142.8 Å) and belong to the space group $P6_1$/$P6_5$, with two molecules in the asymmetric unit. The results of a complete diffraction set collected at the ESRF are given in Table IV below, by comparison with the data previously obtained with soluble PBP2x* (Dessen et al., J. Biol. Chem., 2001, 48, 45106-45112; Gordon et al., J. Mol. Biol., 2000, 299, 477-485).

**Table IV: Compared crystallography data for mini-PBP2x and for PBP2x***

| Crystallography data | Mini-PBP2x | PBP2x* (Dessen et al.) | PBP2x* (Gordon et al.) |
|---|---|---|---|
| Dimensions (Å) | a = b = 136.4, c = 142.8 | a = b = 146.56, c = 132.61 | a = b = 129.9, c = 139.86 |
| Space group | $P6_1$/$P6_5$ | $P3_2$ | $P4_12_12$ |
| Minimum resolution (Å) | 2.5 | 3.2 | 2.4 |
| Number of unique reflexions | 51 815 | 52 413 | 42 234 |
| Rsym (%) | 10 (29.6)* | 11.1 (36.7)* | 6.3 (36.8)* |
| I/σI | 2.3 | 10.2 (3.1)* | |

(continued)

| Crystallography data | Mini-PBP2x | PBP2x* (Dessen et al.) | PBP2x* (Gordon et al.) |
|---|---|---|---|
| Redundancy | 4.0 | 3.2 | 7 |
| Completion (%) | 99.9 | 96.1 (97.8)* | 99.7 (93.5)* |
| *Corresponding value in the final layer of diffraction. | | | |

Table IV shows that the diffraction data from mini-PBP2x amount to 51 815 unique reflexions with an Rsym of 10% (30% in the final layer of diffraction: 2.64 to 2.5 Å), with a degree of completion of 99.9%.

A finer analysis of the data obtained with the mini-PBP2x, as a function of the resolution, is given in Table V below; the most significant data are indicated in bold.

**Table V: Statistical analysis of the diffraction data for mini-PBP2x as a function of resolution**

| Dmin[1] | RfaC[2] | Rfull[3] | Rcum[4] | Av-I[5] | $\sigma$[6] | I/$\sigma$ | Nmea[7] | Nref[8] |
|---|---|---|---|---|---|---|---|---|
| 7.91 | 0.069 | 0.070 | 0.069 | 39 616 | 5 195.0 | 7.6 | 4 938 | 1 556 |
| 5.59 | 0.085 | 0.080 | 0.077 | 18 912 | 2 986.4 | 6.3 | 10 812 | 3 027 |
| 4.56 | 0.092 | 0.075 | 0.085 | 26 060 | 4 275.6 | 6.1 | 14 633 | 3 889 |
| 3.95 | 0.087 | 0.056 | 0.086 | 26 842 | 4 120.7 | 6.5 | 18 118 | 4 617 |
| 3.54 | 0.084 | 0.063 | 0.085 | 16 584 | 2 210.7 | 7.5 | 20 977 | 5 210 |
| 3.23 | 0.094 | 0.067 | 0.086 | 9 999 | 1 434.4 | 7.0 | 23 488 | 5 776 |
| 2.99 | 0.125 | 0.105 | 0.089 | 5 454 | 996.0 | 5.5 | 25 470 | 6 242 |
| 2.80 | 0.165 | 0.136 | 0.092 | 3 227 | 771.4 | 4.2 | 27 395 | 6 710 |
| 2.64 | 0.223 | 0.179 | 0.096 | 2 006 | 648.9 | 3.1 | 29 137 | 7 145 |
| 2.5 | 0.296 | 0.220 | 0.100 | 1 378 | 594.1 | 2.3 | 30 891 | 7 580 |
| 1: minimum resolution (Å), 2: Confidence factor in the range, 3: Confidence factor for full reflexions on a film, 4: Cumulative confidence factor, 5: Mean of intensities <I>, 6: Standard deviation of intensities, 7: Number of reflexions measured, 8: Number of unique reflexions in the resolution range. | | | | | | | | |

The data given in Tables IV and V above show that the mini-PBP2x makes it possible to obtain crystals with a good diffraction power (2.5 Å).

## EXAMPLE 5: SCREENING OF ANTIBIOTIC MOLECULES USING A MINI-PBP2x

The screening of inhibitors of a mini-PBP2x, which can be used as antibiotics, is carried out using binding assays or using assays for inhibition of the enzymatic activity (hydrolytic activity) of the mini-PBP2x, in the presence of the test molecule.

### 1) Mini-PBP2x-binding assay

It has been shown that the intrinsic fluorescence of PBP2x decreases during the binding, at the active site, of inhibitors such as β-lactams (Jamin et al., Biochem. J. 292, 735-741). Consequently, mini-PBP2x inhibitors which can be used as antibiotics are screened in a similar way.

More precisely, the mini-PBP2x, prepared as described in Example 1, is distributed into the wells of a plate (2 to 500 µl of mini-PBP2x in 10 mM phosphate buffer, pH 7), and the test molecules are then added and the plates are incubated at a temperature of between 5°C and 40°C for a period of between 30 s and 1 h. The variation in fluorescence of the mini-PBP2x, in the presence or absence of test molecule, is measured in a wavelength window of between 305 and 360 nm, after excitation at a wavelength of 280 nm; said measurement is made either continuously or after various incubation times of between 30 s and 1 h. The molecules capable of binding at the active site of the mini-PBP2x, corresponding to those for which a decrease in the intrinsic fluorescence of the mini-PBP2x is observed, are selected.

**2) Assay for inhibition of the enzymatic activity of the mini-PBP2x**

The hydrolysis of pseudosubstrates of thioester type [N-benzoyl-D-alanylmercaptoacetic thiol ester (S2d) and carboxymethylbenzoylaminothioacetate thiol ester (S2a)] is measured with the mini-PBP2x, alone or in the presence of test molecule, according to the protocols as described in Zhao et al., mentioned above. The molecules capable of inhibiting the hydrolytic activity of the mini-PBP2x are selected.

**Table V: Statistical analysis of the diffraction data for mini-PBP2x as a function of resolution**

| Dmin[1] | Rfac[2] | Rfull[3] | Rcum[4] | Av-I[5] | $\sigma$[6] | I/$\sigma$ | Nmea[7] | Nref[8] |
|---|---|---|---|---|---|---|---|---|
| 7.91 | 0.069 | 0.070 | 0.069 | 39 616 | 5 195.0 | 7.6 | 4 938 | 1 556 |
| 5.59 | 0.085 | 0.080 | 0.077 | 18 912 | 2 986.4 | 6.3 | 10 812 | 3 027 |
| 4.56 | 0.092 | 0.075 | 0.085 | 26 060 | 4 275.6 | 6.1 | 14 633 | 3 889 |
| 3.95 | 0.087 | 0.056 | 0.086 | 26 842 | 4 120.7 | 6.5 | 18 118 | 4 617 |
| 3.54 | 0.084 | 0.063 | 0.085 | 16 584 | 2 210.7 | 7.5 | 20 977 | 5 210 |
| 3.23 | 0.094 | 0.067 | 0.086 | 9 999 | 1 434.4 | 7.0 | 23 488 | 5 776 |
| 2.99 | 0.125 | 0.105 | 0.089 | 5 454 | 996.0 | 5.5 | 25 470 | 6 242 |
| 2.80 | 0.165 | 0.136 | 0.092 | 3 227 | 771.4 | 4.2 | 27 395 | 6 710 |
| 2.64 | 0.223 | 0.179 | 0.096 | 2 006 | 648.9 | 3.1 | 29 137 | 7 145 |
| 2.5 | 0.296 | 0.220 | 0.100 | 1 378 | 594.1 | 2.3 | 30 891 | 7 580 |

1: minimum resolution (Å), 2: Confidence factor in the range,
3: Confidence factor for full reflexions on a film,
4: Cumulative confidence factor, 5: Mean of intensities <I>,
6: Standard deviation of intensities, 7: Number of reflexions measured, 8: Number of unique reflexions in the resolution range.

The data given in Tables IV and V above show that the mini-PBP2x makes it possible to obtain crystals with a good diffraction power (2.5 Å).

## EXAMPLE 5: SCREENING OF ANTIBIOTIC MOLECULES USING A MINI-PBP2x

The screening of inhibitors of a mini-PBP2x, which can be used as antibiotics, is carried out using binding assays or using assays for inhibition of the enzymatic activity (hydrolytic activity) of the mini-PBP2x, in the presence of the test molecule.

**1) Mini-PBP2x-binding assay**

It has been shown that the intrinsic fluorescence of PBP2x decreases during the binding, at the active site, of inhibitors such as β-lactams (Jamin et al., Biochem. J. 292, 735-741). Consequently, mini-PBP2x inhibitors which can be used as antibiotics are screened in a similar way.

More precisely, the mini-PBP2x, prepared as described in Example 1, is distributed into the wells of a plate (2 to 500 μl of mini-PBP2x in 10 mM phosphate buffer, pH 7), and the test molecules are then added and the plates are incubated at a temperature of between 5°C and 40°C for a period of between 30 s and 1 h. The variation in fluorescence of the mini-PBP2x, in the presence or absence of test molecule, is measured in a wavelength window of between 305 and 360 nm, after excitation at a wavelength of 280 nm; said measurement is made either continuously or after various incubation times of between 30 s and 1 h. The molecules capable of binding at the active site of the mini-PBP2x, corresponding to those for which a decrease in the intrinsic fluorescence of the mini-PBP2x is observed, are selected.

**2) Assay for inhibition of the enzymatic activity of the mini-PBP2x**

The hydrolysis of pseudosubstrates of thioester type [N-benzoyl-D-alanylmercaptoacetic thiol ester (S2d) and carboxymethylbenzoylaminothioacetate thiol ester (S2a)] is measured with the mini-PBP2x, alone or in the presence of test molecule, according to the protocols as described in Zhao et al., mentioned above. The molecules capable of inhibiting the hydrolytic activity of the mini-PBP2x are selected.

## Claims

1. Protein, named mini-PBP2X, derived from a *Streptococcus pneumoniae* PBP2x, **characterized in that** it consists of a concatenation of the fragments corresponding respectively to the amino acids located between positions 74 to 90, 186 to 199, 218 to 228 and 257-750, with reference to the sequence of the PBP2x protein of the strain R6 (SWISSPROT P14677 or GENBANK 18266817), each one of said fragments being preceded by a peptide fragment of 1 to 7 amino acids.

2. Protein according to Claim 1, **characterized in that** said peptide fragment comprises amino acids of said *Streptococcus pneumoniae* PBP2x protein located between positions -1 to -7, relative to the residues at positions 74, 186, 218 and 257, and/or between positions +1 to +7, relative to the residues at positions 90, 199 and 228, as defined in Claim 1.

3. Protein according to Claim 1 or Claim 2, **characterized in that** said peptide fragment comprises amino acids chosen from alanine (A), serine (S), glycine (G) and threonine (T).

4. Protein according to any one of Claims 1 to 3, **characterized in that** it is derived from a β-lactam-resistant strain of *S. pneumoniae*.

5. Protein according to any one of Claims 1 to 3, **characterized in that** it has the sequence SEQ ID No. 1.

6. Protein according to any one of Claims 1 to 5, **characterized in that** it comprises a substitution of at least one methionine residue with a selenomethionine residue.

7. Protein according to any one of Claims 1 to 6, **characterized in that** it is associated with a ligand.

8. Protein according to any one of Claims 1 to 7, **characterized in that** it is in the form of a crystal.

9. Isolated nucleic acid molecule, **characterized in that** it has a sequence encoding the mini-PBP2x according to any one of Claims 1 to 6, which are sense or antisense.

10. Recombinant vector, **characterized in that** it comprises the nucleic acid molecule according to Claim 9.

11. Recombinant vector according to Claim 10, **characterized in that** it consists of a prokaryotic vector.

12. Cells transformed with a recombinant vector according to either one of Claims 10 and 11.

13. Cells according to Claim 12, **characterized in that** they are prokaryotic cells.

14. Use of a mini-PBP2x according to any one of Claims 1 to 8, for screening antibiotics.

15. Method for screening antibiotics, **characterized in that** it comprises at least the following steps:

$a_1$)bringing a mini-PBP2x according to any one of Claims 1 to 7 into contact with a test substance,
$b_1$)detecting, by any suitable means, the binding of said test molecule with the mini-PBP2x and/or the inhibition of the activity of said mini-PBP2x resulting from this binding, and
$c_1$)selecting and identifying the active substances capable of binding to the mini-PBP2x and/or of inhibiting the activity of said mini-PBP2x, which can be used as antibiotics.

16. Method for identifying antibiotics, **characterized in that** it comprises at least the following steps:

$a_2$)preparing crystals from a mini-PBP2x according to any one of Claims 1 to 7,
$b_2$)determining the three-dimensional structure of said mini-PBP2x from the crystal obtained in $a_2$), and
$c_2$)identifying active substances capable of binding to the mini-PBP2x and/or of inhibiting the activity of said mini-PBP2x, which can be used as antibiotics.

17. Screening kit for implementing the method according to Claim 15 or Claim 16, **characterized in that** it includes at least one protein, according to any one of Claims 1 to 8.

**Patentansprüche**

1. Protein mit der Bezeichnung Mini-PBP2x, das sich von einem *Streptococcus pneumoniae-PBP2x* ableitet, **dadurch gekennzeichnet, dass** es aus einer Aneinanderkettung der Fragmente besteht, die den Aminosäuren entsprechen, die sich zwischen Position 74 bis 90, 186 bis 199, 218 bis 228 bzw. 257 bis 750 befinden, bezogen auf die Sequenz des PBP2x-Proteins des Stamms R6 (SWISSPROT P14677 oder GENBANK 18266817), wobei jedem dieser Fragmente ein Peptidfragment mit 1 bis 7 Aminosäuren vorausgeht.

**2.** Protein nach Anspruch 1, **dadurch gekennzeichnet, dass** das Peptidfragment Aminosäuren des PBP2x-Proteins von *Streptococcus pneumoniae* umfasst, die sich bezüglich der Reste an Position 74, 186, 218 und 257 zwischen Position -1 bis -7 und/oder bezüglich der Reste an Position 90, 199 und 228 zwischen Position +1 bis +7 befinden, wie in Anspruch 1 definiert.

**3.** Protein nach Anspruch 1 oder Anspruch 2, **dadurch gekennzeichnet, dass** das Peptidfragment Aminosäuren umfasst, die ausgewählt sind aus Alanin (A), Serin (S), Glycin (G) und Threonin (T).

**4.** Protein nach einem der Ansprüche 1 bis 3, **dadurch gekennzeichnet, dass** es sich von einem β-Lactam-resistenten Stamm von *S. pneumoniae* ableitet.

**5.** Protein nach einem der Ansprüche 1 bis 3, **dadurch gekennzeichnet, dass** es die Sequenz SEQ ID NO:1 hat.

**6.** Protein nach einem der Ansprüche 1 bis 5, **dadurch gekennzeichnet, dass** es eine Substitution von wenigstens einem Methioninrest durch einen Selenomethioninrest umfasst.

**7.** Protein nach einem der Ansprüche 1 bis 6, **dadurch gekennzeichnet, dass** es mit einem Liganden assoziiert ist.

**8.** Protein nach einem der Ansprüche 1 bis 7, **dadurch gekennzeichnet, dass** es in Form eines Kristalls vorliegt.

**9.** Isoliertes Nukleinsäuremolekül, **dadurch gekennzeichnet, dass** es eine Sequenz hat, die das Mini-PBP2x nach einem der Ansprüche 1 bis 6 codiert, die Sense oder Antisense ist.

**10.** Rekombinanter Vektor, **dadurch gekennzeichnet, dass** er das Nukleinsäuremolekül nach Anspruch 9 umfasst.

**11.** Rekombinanter Vektor nach Anspruch 10, **dadurch gekennzeichnet, dass** er aus einem prokaryontischen Vektor besteht.

**12.** Zellen, transformiert mit einem rekombinanten Vektor nach irgendeinem der Ansprüche 10 und 11.

**13.** Zellen nach Anspruch 12, **dadurch gekennzeichnet, dass** es Prokaryontenzellen sind.

**14.** Verwendung eines Mini-PBP2x nach einem der Ansprüche 1 bis 8 zum Screening von Antibiotika.

**15.** Verfahren zum Screening von Antibiotika, **dadurch gekennzeichnet, dass** es wenigstens die folgenden Schritte umfasst:

$a_1$) Inkontaktbringen eines Mini-PBP2x nach einem der Ansprüche 1 bis 7 mit einer Testsubstanz,
$b_1$) Nachweisen, mit einem geeigneten Mittel, der Bindung des Testmoleküls mit dem Mini-PBP2x und/oder der Hemmung der Aktivität des Mini-PBP2x als Folge dieser Bindung, und
$c_1$) Selektionieren und Identifizieren der aktiven Substanzen, die in der Lage sind, an das Mini-PBP2x zu binden und/oder die Aktivität des Mini-PBP2x zu hemmen, die als Antibiotika verwendet werden können.

**16.** Verfahren zum Identifizieren von Antibiotika, **dadurch gekennzeichnet, dass** es wenigstens die folgenden Schritte umfasst:

$a_2$) Herstellen von Kristallen eines Mini-PBP2x nach einem der Ansprüche 1 bis 7,
$b_2$) Bestimmen der dreidimensionalen Struktur des Mini-PBP2x aus dem in $a_2$) erhaltenen Kristall, und
$c_2$) Identifizieren aktiver Substanzen, die in der Lage sind, an das Mini-PBP2x zu binden und/oder die Aktivität des Mini-PBP2x zu hemmen, die als Antibiotika verwendet werden können.

**17.** Screening-Kit zur Durchführung des Verfahrens nach Anspruch 15 oder Anspruch 16, **dadurch gekennzeichnet, dass** er wenigstens ein Protein nach einem der Ansprüche 1 bis 8 enthält.

**Revendications**

**1.** Protéine, nommée mini-PBP2X, dérivée de *Streptococcus pneumoniae* PBP2x, **caractérisée en ce qu'**elle est composée par une concaténation des fragments correspondant respectivement aux acides aminés situés entre les positions 74 à 90, 186 à 199, 218 à 228 et 257-750, par référence à la séquence de la protéine PBP2x de la souche R6 (SWISSPROT P14677 ou GENBANK 18266817), chacun desdits fragments étant précédé d'un fragment peptidique de 1 à 7 acides aminés.

**2.** Protéine, selon la revendication 1,
**caractérisée en ce que** ledit fragment peptidique comprend des acides aminés de ladite protéine de *Streptococcus pneumoniae* PBP2x situés entre les positions -1 à -7, par rapport aux résidus situés aux positions 74, 186, 218 et 257, et/ou entre les positions +1 à +7, par rapport aux résidus situés aux positions 90, 199 et 228, comme cela est défini à la revendication 1.

**3.** Protéine, selon la revendication 1 ou la revendication 2, **caractérisée en ce que** ledit fragment peptidique comprend des acides aminés choisis à partir de l'alanine (A), la sérine (S), la glycine (G) et la thréonine (T).

**4.** Protéine, selon l'une quelconque des revendications 1 à 3, **caractérisée en ce qu'**elle est dérivée d'une souche de *S. pneumoniae* résistant aux β-lactamines.

**5.** Protéine, selon l'une quelconque des revendications 1 à 3, **caractérisée en ce qu'**elle a la séquence SEQ ID n° 1.

**6.** Protéine, selon l'une quelconque des revendications 1 à 5, **caractérisée en ce qu'**elle comprend une substitution d'au moins un résidu de méthionine par un résidu de sélénométhionine.

**7.** Protéine, selon l'une quelconque des revendications 1 à 6, **caractérisée en ce qu'**elle est associée à un ligand.

**8.** Protéine, selon l'une quelconque des revendications 1 à 7, **caractérisée en ce qu'**elle est sous forme de cristal.

**9.** Molécule isolée d'acide nucléique, **caractérisée en ce qu'**elle a une séquence codant pour la mini-PBP2x, selon l'une quelconque des revendications 1 à 6, qui est sens ou antisens.

**10.** Vecteur recombinant, **caractérisé en ce qu'**il comprend la molécule d'acide nucléique selon la revendication 9.

**11.** Vecteur recombinant, selon la revendication 10,
**caractérisé en ce qu'**il se compose d'un vecteur procaryote.

**12.** Cellules transformées avec un vecteur recombinant selon l'une ou l'autre des revendications 10 et 11.

**13.** Cellules, selon la revendication 12,
**caractérisées en ce que** ce sont des cellules procaryotes.

**14.** Utilisation d'une mini-PBP2x, selon l'une quelconque des revendications 1 à 8, pour effectuer un criblage d'antibiotiques.

**15.** Procédé de criblage d'antibiotiques,
**caractérisé en ce qu'**il comprend au moins les étapes suivantes:

$a_1$) mettre en contact une mini-PBP2x, selon l'une quelconque des revendications 1 à 7, avec une substance à tester ;
$b_1$) détecter, par n'importe quel moyen approprié, la liaison de ladite molécule à tester avec la mini-PBP2x et/ou l'inhibition de l'activité de ladite mini-PBP2x résultant de cette liaison ; et
$c_1$) sélectionner et identifier les substances actives capables de se lier à la mini-PBP2x et/ou d'inhiber l'activité de ladite mini-PBP2x, lesquelles peuvent être utilisées comme antibiotiques.

**16.** Procédé d'identification d'antibiotiques,
**caractérisé en ce qu'**il comprend au moins les étapes suivantes:

$a_2$) préparer des cristaux à partir d'une mini-PBP2x, selon l'une quelconque des revendications 1 à 7 ;
$b_2$) déterminer la structure tridimensionnelle de ladite mini-PBP2x à partir du cristal obtenu au paragraphe $a_2$) ; et
$c_2$) identifier des substances actives capables de se lier à la mini-PBP2x et/ou d'inhiber l'activité de ladite mini-PBP2x, lesquelles peuvent être utilisées comme antibiotiques.

**17.** Trousse pour criblage destinée à mettre en oeuvre le procédé, selon la revendication 15 ou la revendication 16, **caractérisée en ce qu'**elle comprend au moins une protéine selon l'une quelconque des revendications 1 à 8.

```
PBP2x    1   M K W T K R V I R Y A T K N R K S P A E N R R R V G K S L S   30
MINI-2x   1   - - - - - - - - - - - - - - - - - - - - - - - - - - - - - -   0

PBP2x   31   L L S V F V F A I F L V N F A V I I G T G T R F G T D L A K   60
MINI-2x   1   - - - - - - - - - - - - - - - - - - - - - - - - - - - - - -   0

PBP2x   61   E A K K V H Q T T R T V P A K R G T I Y D R N G V P I A E D   90
MINI-2x   1   - - - - - - - - - - G S G A K R G T I Y D R N G V P I A E D   20

PBP2x   91   A T S Y N V Y A V I D E N Y K S A T G K I L Y V E K T Q F N   120
MINI-2x  21   A T S G G - - - - - - - - - - - - - - - - - - - - - - - - -   25

PBP2x  121   K V A E V F H K Y L D M E E S Y V R E Q L S Q P N L K Q V S   150
MINI-2x  26   - - - - - - - - - - - - - - - - - - - - - - - - - - - - - -   25

PBP2x  151   F G A K G N G I T Y A N M M S I K K E L E A A E V K G I D F   180
MINI-2x  26   - - - - - - - - - - - - - - - - - - - - - - - - - - - - - -   25

PBP2x  181   T T S P N R S Y P N G Q F A S S F I G L A Q L H E N E D G S   210
MINI-2x  26   - - - P N R S Y P N G Q F A S S F I G G - - - - - - - - - -   42

PBP2x  211   K S L L G T S G M E S S L N S I L A G T D G I I T Y E K D R   240
MINI-2x  43   - - - - - - - G M E S S L N S I L A G G G G - - - - - - - -   57

PBP2x  241   L G N I V P G T E Q V S Q R T M D G K D V Y T T I S S P L Q   270
MINI-2x  58   - - - - - - - - - - - - - - - D G K D V Y T T I S S P L Q   71

PBP2x  271   S F M E T Q M D A F Q E K V K G K Y M T A T L V S A K T G E   300
MINI-2x  72   S F M E T Q M D A F Q E K V K G K Y M T A T L V S A K T G E   101

PBP2x  301   I L A T T Q R P T F D A D T K E G I T E D F V W R D I L Y Q   330
MINI-2x 102   I L A T T Q R P T F D A D T K E G I T E D F V W R D I L Y Q   131

PBP2x  331   S N Y E P G S T M K V M M L A A A I D N N T F P G G E V F N   360
MINI-2x 132   S N Y E P G S T M K V M M L A A A I D N N T F P G G E V F N   161

PBP2x  361   S S E L K I A D A T I R D W D V N E G L T G G R M M T F S Q   390
MINI-2x 162   S S E L K I A D A T I R D W D V N E G L T G G R M M T F S Q   191

PBP2x  391   G F A H S S N V G M T L L E Q K M G D A T W L D Y L N R F K   420
MINI-2x 192   G E A H S S N V G M T L L E Q K M G D A T W L D Y L N R F K   221
```

FIGURE 1

22

```
PBP2x    421  F G V P T R F G L T D E Y A G Q L P A D N I V N I A Q S S F  450
MINI-2x  222  F G V P T R F G L T D E Y A G Q L P A D N I V N I A Q S S F  251

PBP2x    451  G Q G I S V T Q T Q M I R A F T A I A N D G V M L E P K F I  480
MINI-2x  252  G Q G I S V T Q T Q M I R A F T A I A N D G V M L E P K F I  281

PBP2x    481  S A I Y D P N D Q T A R K S Q K E I V G N P V S K D A A S L  510
MINI-2x  282  S A I Y D P N D Q T A R K S Q K E I V G N P V S K D A A S L  311

PBP2x    511  T R T N M V L V G T D P V Y G T M Y N H S T G K P T V T V P  540
MINI-2x  312  T R T N M V L V G T D P V Y G T M Y N H S T G K P T V T V P  341

PBP2x    541  G Q N V A L K S G T A Q I A D E K N G G Y L V G L T D Y I F  570
MINI-2x  342  G Q N V A L K S G T A Q I A D E K N G G Y L V G L T D Y I F  371

PBP2x    571  S A V S M S P A E N P D F I L Y V T V Q Q P E H Y S G I Q L  600
MINI-2x  372  S A V S M S P A E N P D F I L Y V T V Q Q P E H Y S G I Q L  401

PBP2x    601  G E F A N P I L E R A S A M K D S L N L Q T T A K A L E Q V  630
MINI-2x  402  G E F A N P I L E R A S A M K D S L N L Q T T A K A L E Q V  431

PBP2x    631  S Q Q S P Y P M P S V K D I S P G D L A E E L R R N L V Q P  660
MINI-2x  432  S Q Q S P Y P M P S V K D I S P G D L A E E L R R N L V Q P  461

PBP2x    661  I V V G T G T K I K N S S A E E G K N L A P N Q Q V L I L S  690
MINI-2x  462  I V V G T G T K I K N S S A E E G K N L A P N Q Q V L I L S  491

PBP2x    691  D K A E E V P D M Y G W T K E T A E T L A K W L N I E L E F  720
MINI-2x  492  D K A E E V P D M Y G W T K E T A E T L A K W L N I E L E F  521

PBP2x    721  Q G S G S T V Q K Q D V R A N T A I K D I K K I T L T L G D  750
MINI-2x  522  Q G S G S T V Q K Q D V R A N T A I K D I K K I T L T L G D  551

PBP2x    750                                                             750
MINI-2x  551                                                             551
```

FIGURE 1 (continued)

**EP 1 521 771 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DOERN et al.** *Antimicrob. Agents Chemother,* 2001, vol. 45, 1721-1729
- **HAKENBECK et al.** *J. Bacteriol.,* 1998, vol. 180, 1831-1840
- **MOUZ et al.** *P.N.A.S.,* 1998, vol. 95, 13403-13406
- *J. Biol. Chem.,* 1999, vol. 274, 19175-19180
- **PARES et al.** *Nature Struct. Biol.,* 1996, vol. 3, 284-289
- **GORDON et al.** *J. Mol. Biol.,* 2000, vol. 299, 477-485
- **DESSEN et al.** *J. Biol. Chem.,* 2001, vol. 276, 45106-45112
- **GORDON et al.** *J. Mol. Biol.,* 2000, vol. 299, 477-485
- **LAIBLE et al.** *Mol. Microbiol.,* 1989, vol. 3, 1337-1348
- **FREDERICK M. AUSUBEL.** Current Protocols in Molecular Biology. Wiley and son Inc, 2000
- **KUNKEL et al.** *P.N.A.S.,* 1985, vol. 82, 488-492
- **JAMIN et al.** *Biochem. J.,* 1993, vol. 292, 735-741
- **ZHAO et al.** *J. Bacteriol.,* 1997, vol. 179, 4901-4908
- **MOUZ et al.** *J. Biol. Chem.,* 1999, vol. 274, 19175-19180
- **MOUZ et al.** *P.N.A.S.,* 1998, vol. 95, 13403-13406
- **ADAM et al.** *Biochem. J.,* 1990, vol. 270, 525-529
- *Acta Crystallograph. Sect. D,* 1994, vol. 54, 905-921
- **DESSEN et al.** *J. Biol. Chem.,* 2001, vol. 48, 45106-45112
- **JAMIN et al.** *Biochem. J,* vol. 292, 735-741

24

SEQUENCE LISTING

<110> COMMISSARIAT A L'ENERGIE ATOMIQUE
CENTRE NATIONAL DE LA RECHERCHE SCIENTIFIQUE
DIDEBERG Otto
VERNET Thierry
MOUZ Nicolas

<120> STREPTOCOCCUS PNEUMONIAE PBP2x MINI-PROTEIN AND USES THEREOF.

<130> F263FR79s

<140>
<141>

<160> 9

<170> PatentIn Ver. 2.1

<210> 1
<211> 551
<212> PRT
<213> Streptococcus pneumoniae

<400> 1

```
Gly Ser Gly Ala Lys Arg Gly Thr Ile Tyr Asp Arg Asn Gly Val Pro
 1               5               10                  15

Ile Ala Glu Asp Ala Thr Ser Gly Gly Pro Asn Arg Ser Tyr Pro Asn
            20              25              30

Gly Gln Phe Ala Ser Ser Phe Ile Gly Gly Gly Met Glu Ser Ser Leu
        35              40              45

Asn Ser Ile Leu Ala Gly Gly Gly Gly Asp Gly Lys Asp Val Tyr Thr
    50              55              60

Thr Ile Ser Ser Pro Leu Gln Ser Phe Met Glu Thr Gln Met Asp Ala
65              70              75              80

Phe Gln Glu Lys Val Lys Gly Lys Tyr Met Thr Ala Thr Leu Val Ser
            85              90              95

Ala Lys Thr Gly Glu Ile Leu Ala Thr Thr Gln Arg Pro Thr Phe Asp
            100             105             110

Ala Asp Thr Lys Glu Gly Ile Thr Glu Asp Phe Val Trp Arg Asp Ile
        115             120             125

Leu Tyr Gln Ser Asn Tyr Glu Pro Gly Ser Thr Met Lys Val Met Met
    130             135             140

Leu Ala Ala Ala Ile Asp Asn Asn Thr Phe Pro Gly Gly Glu Val Phe
145             150             155             160

Asn Ser Ser Glu Leu Lys Ile Ala Asp Ala Thr Ile Arg Asp Trp Asp
            165             170             175
```

```
Val Asn Glu Gly Leu Thr Gly Gly Arg Thr Met Thr Phe Ser Gln Gly
        180             185             190

Phe Ala His Ser Ser Asn Val Gly Met Thr Leu Leu Glu Gln Lys Met
        195             200             205

Gly Asp Ala Thr Trp Leu Asp Tyr Leu Asn Arg Phe Lys Phe Gly Val
        210             215             220

Pro Thr Arg Phe Gly Leu Thr Asp Glu Tyr Ala Gly Gln Leu Pro Ala
225             230             235             240

Asp Asn Ile Val Asn Ile Ala Gln Ser Ser Phe Gly Gln Gly Ile Ser
            245             250             255

Val Thr Gln Thr Gln Met Ile Arg Ala Phe Thr Ala Ile Ala Asn Asp
        260             265             270

Gly Val Met Leu Glu Pro Lys Phe Ile Ser Ala Ile Tyr Asp Pro Asn
        275             280             285

Asp Gln Thr Ala Arg Lys Ser Gln Lys Glu Ile Val Gly Asn Pro Val
        290             295             300

Ser Lys Asp Ala Ala Ser Leu Thr Arg Thr Asn Met Val Leu Val Gly
305             310             315             320

Thr Asp Pro Val Tyr Gly Thr Met Tyr Asn His Ser Thr Gly Lys Pro
            325             330             335

Thr Val Thr Val Pro Gly Gln Asn Val Ala Leu Lys Ser Gly Thr Ala
            340             345             350

Gln Ile Ala Asp Glu Lys Asn Gly Gly Tyr Leu Val Gly Leu Thr Asp
        355             360             365

Tyr Ile Phe Ser Ala Val Ser Met Ser Pro Ala Glu Asn Pro Asp Phe
370             375             380

Ile Leu Tyr Val Thr Val Gln Gln Pro Glu His Tyr Ser Gly Ile Gln
385             390             395             400

Leu Gly Glu Phe Ala Asn Pro Ile Leu Glu Arg Ala Ser Ala Met Lys
            405             410             415

Asp Ser Leu Asn Leu Gln Thr Thr Ala Lys Ala Leu Glu Gln Val Ser
            420             425             430

Gln Gln Ser Pro Tyr Pro Met Pro Ser Val Lys Asp Ile Ser Pro Gly
            435             440             445

Asp Leu Ala Glu Glu Leu Arg Arg Asn Leu Val Gln Pro Ile Val Val
450             455             460

Gly Thr Gly Thr Lys Ile Lys Asn Ser Ser Ala Glu Glu Gly Lys Asn
465             470             475             480
```

15

```
Leu Ala Pro Asn Gln Gln Val Leu Ile Leu Ser Asp Lys Ala Glu Glu
                485             490             495

Val Pro Asp Met Tyr Gly Trp Thr Lys Glu Thr Ala Glu Thr Leu Ala
                500             505             510

Lys Trp Leu Asn Ile Glu Leu Glu Phe Gln Gly Ser Gly Ser Thr Val
        515             520             525

Gln Lys Gln Asp Val Arg Ala Asn Thr Ala Ile Lys Asp Ile Lys Lys
        530             535             540

Ile Thr Leu Thr Leu Gly Asp
545             550
```

<210> 2
<211> 46
<212> DNA
<213> Artificial sequence

<220>
<223> Description of the artificial sequence:primer

<400> 2
gtcgacttag tctcctaaag ttaatttaat ttttttaatg tttttg 46

<210> 3
<211> 21
<212> DNA
<213> Artificial sequence

<220>
<223> Description of the artificial sequence:primer

<400> 3
ggatccggga caggcactcg c 21

<210> 4
<211> 43
<212> DNA
<213> Artificial sequence

<220>
<223> Description of the artificial sequence:primer

<400> 4
cataaatagt cccacgtttg gccccggatc cacgcggaac cag 43

<210> 5
<211> 51
<212> DNA
<213> Artificial sequence

<220>
<223> Description of the artificial sequence:primer

<400> 5
gtttgggtaa ctacgattgg gacctccaga ggttgcatcc tcagcaatcg g  51

<210> 6
<211> 48
<212> DNA
<213> Artificial sequence

<220>
<223> Description of the artificial sequence:primer

<400> 6
gttcaaggaa ctctccattc caccgccgat aaaactagaa gcaaattg  48

<210> 7
<211> 49
<212> DNA
<213> Artificial sequence

<220>
<223> Description of the artificial sequence:primer

<400> 7
tgtataaaca tccttaccgt ccccacctcc ccctgcaaga atactgttc  49

<210> 8
<211> 30
<212> DNA
<213> Artificial sequence

<220>
<223> Description of the artificial sequence:primer

<400> 8
ccgcatatgg ccaaacgtgg gactatttat  30

<210> 9
<211> 32
<212> DNA
<213> Artificial sequence

<220>
<223> Description of the artificial séquence:primer

<400> 9
ggctcgagtt agtctcctaa agttaatgta at  32